# EUROPEAN PATENT SPECIFICATION

(11) **EP 2 214 598 B1**
(45) Date of publication and mention of the grant of the patent: **18.05.2016**
(21) Application number: 08842259.7
(22) Date of filing: 22.10.2008
(51) Int. Cl.: A61F 2/82, C08G 63/08

(54) **RANDOM AMORPHOUS TERPOLYMER CONTAINING LACTIDE AND GLYCOLIDE**
AMORPHES RANDOM-TERPOLYMER MIT LAKTID UND GLYKOLID
TERPOLYMÈRE AMORPHE ALÉATOIRE CONTENANT DU LACTIDE ET DU GLYCOLIDE

(30) Priority: 23.10.2007 US 877622
(43) Date of publication of application: 11.08.2010
(73) Proprietor: Abbott Cardiovascular Systems Inc., Santa Clara, CA 95054-2807 (US)
(72) Inventor: PACETTI, Stephen, D., San Jose CA 95130 (US); TROLLSAS, Mikael, O., San Jose CA 95124 (US); LIM, Florencia, Union City CA 94587 (US)
(74) Representative: ZBM Patents - Zea, Barlocci & Markvardsen
(86) International application number: PCT/US2008/080719
(87) International publication number: WO 2009/055426

(56) References cited:
- US-A- 5 076 807
- US-A- 5 085 629
- US-A- 6 165 202
- US-A1- 2005 079 202
- US-A1- 2005 112 171

## Description

### FIELD OF THE INVENTION

The present invention relates to bioabsorbable amorphous polymers for controlling the release of a drug from a coating on an implantable device.

### BACKGROUND OF THE INVENTION

Percutaneous coronary intervention (PCI) is a procedure for treating heart disease. A catheter assembly having a balloon portion is introduced percutaneously into the cardiovascular system of a patient via the radial, brachial or femoral artery. The catheter assembly is advanced through the coronary vasculature until the balloon portion is positioned across the occlusive lesion. Once in position across the lesion, the balloon is inflated to a predetermined size to radially compress the atherosclerotic plaque of the lesion to remodel the lumen wall. The balloon is then deflated to a smaller profile to allow the catheter to be withdrawn from the patient's vasculature.

Problems associated with the above procedure include formation of intimal flaps or torn arterial linings which can collapse and occlude the blood conduit after the balloon is deflated. Moreover, thrombosis and restenosis of the artery may develop over several months after the procedure, which may require another angioplasty procedure or a surgical by-pass operation. To reduce the partial or total occlusion of the artery by the collapse of the arterial lining and to reduce the chance of thrombosis or restenosis, a stent is implanted in the artery to keep the artery open.

Drug delivery stents have reduced the incidence of in-stent restenosis (ISR) after PCI (see, e.g., Serruys, P.W., et al., J. Am. Coll. Cardiol. 39:393-399 (2002)), which has plagued interventional cardiology for more than a decade. However, a few challenges remain in the art of drug delivery stents. For example, release of a drug from a coating formed of an amorphous may often have a burst release of the drug, resulting in insufficient control release of the drug.

Therefore, there is a need for a coating that provides for a controlled release of a drug in the coating.

The embodiments of the present invention address the above-identified needs and issues.

### SUMMARY OF THE INVENTION

The present invention provides a coating on an implantable device that comprises a bioabsorbable random amorphous terpolymer according to claim 1, for controlling the release of a drug from the coating. The terpolymer comprises units derived from lactide and glycolide and units derived from a third monomer. The glycolide provides an accelerated or enhanced degradation of the terpolymer. The lactide monomer provides mechanical strength to the terpolymer. The third monomer provides beneficial properties to the terpolymer, e.g., lowering the overall polymer glass transition temperature (Tg) so as to enhance drug permeability, water permeability, and enhancing degradation rate of the polymer, imparting greater flexibility and elongation, and improving mechanical properties of a coating formed of the terpolymer. A requisite attribute of the third monomer is that, if a homopolymer were to be formed of the third monomer, the homopolymer would have a Tg below about -20 °C.

A coating formed of the terpolymer described herein can degrade within about 1 month, 2 months, 3 months, 4 months, 6 months, 12 months, 18 months, or 24 months after implantation of a medical device comprising the coating. In some embodiments, the coating can completely degrade or fully absorb within 24 months after implantation of a medical device comprising the coating.

In some embodiments, the coating can include one or more other biocompatible polymers, which are described below.

In some embodiments, the terpolymer can form a coating that can include one or more bioactive agents, e.g., drug(s). Some exemplary bioactive agents that can be included in a coating described herein are paclitaxel, docetaxel, estradiol, 17-beta-estradiol, nitric oxide donors, super oxide dismutases, super oxide dismutases mimics, 4-amino-2,2,6,6-tetramethylpiperidine-1-oxyl (4-amino-TEMPO), biolimus, tacrolimus, dexamethasone, dexamethasone acetate, rapamycin, rapamycin derivatives, 40-*O*-(2-hydroxy)ethyl-rapamycin (everolimus), 40-*O*-(3-hydroxy)propyl-rapamycin, 40-*O*-[2-(2-hydroxy)ethoxy]ethyl-rapamycin, 40-*O*-tetrazole-rapamycin, 40-epi-(N1-tetrazolyl)-rapamycin (ABT-578), zotarolimus, Biolimus A9 (Biosensors International, Singapore), AP23572 (Ariad Pharmaceuticals), γ-hiridun, clobetasol, pimecrolimus, imatinib mesylate, midostaurin, feno fibrate, prodrugs thereof, co-drugs thereof, and combinations thereof. Some other examples of the bioactive agent include siRNA and/or other oligoneucleotides that inhibit endothelial cell migration. Some further examples of the bioactive agent can also be lysophosphatidic acid (LPA) or sphingosine-1-phosphate (S1P). LPA is a "bioactive" phospholipid able to generate growth factor-like activities in a wide variety of normal and malignant cell types. LPA plays an important role in normal physiological processes such as wound healing, and in vascular tone, vascular integrity, or reproduction. As used herein, in some embodiments, the term "drug" and the term "bioactive agent" are used interchangeably.

The implantable device described herein can be formed on an implantable device such as a stent, which can be implanted in a patient to treat, prevent, mitigate, or reduce a vascular medical condition, or to provide a pro-healing effect.

In some embodiments, the vascular medical condition or vascular condition is a coronary artery disease (CAD) and/or a peripheral vascular disease (PVD). Some examples of such vascular medical diseases are restenosis and/or atherosclerosis.

Some other examples of these conditions include thrombosis, hemorrhage, vascular dissection or perforation, vascular aneurysm, vulnerable plaque, chronic total occlusion, claudication, anastomotic proliferation (for vein and artificial grafts), bile duct obstruction, urethral obstruction, tumor obstruction, or combinations of these.

### DETAILED DESCRIPTION

The present invention provides a coating on an implantable device that comprises a bioabsorbable random amorphous terpolymer as defined in claim 1, for controlling the release of a drug from the coating. The terpolymer comprises units derived from lactide and glycolide and units derived from a third monomer. The glycolide provides an accelerated or enhanced degradation of the terpolymer. The lactide monomer provides mechanical strength to the terpolymer. The third monomer provides beneficial properties to the terpolymer, e.g., lowering the overall polymer glass transition temperature (Tg) so as to enhance drug permeability, water permeability, and enhancing degradation rate of the polymer, imparting greater flexibility and elongation, and improving mechanical properties of a coating formed of the terpolymer. A requisite attribute of the third monomer is that, if a homopolymer were to be formed of the third monomer, the homopolymer would have a Tg below about -20 °C.

In some embodiments, the coating can include one or more other biocompatible polymers, which are described below.

In some embodiments, the terpolymer can form a coating that can include one or more bioactive agents, e.g., drug(s). Some exemplary bioactive agents that can be included in a coating described above are paclitaxel, docetaxel, estradiol, 17-beta-estradiol, nitric oxide donors, super oxide dismutases, super oxide dismutases mimics, 4-amino-2,2,6,6-tetramethylpiperidine-1-oxyl (4-amino-TEMPO), biolimus, tacrolimus, dexamethasone, dexamethasone acetate, rapamycin, rapamycin derivatives, 40-*O*-(2-hydroxy)ethyl-rapamycin (everolimus), 40-*O*-(3-hydroxy)propyl-rapamycin, 40-*O*-[2-(2-hydroxy)ethoxy]ethyl-rapamycin, and 40-*O*-tetrazole-rapamycin, 40-epi-(N1-tetrazolyl)-rapamycin (ABT-578), zotarolimus, Biolimus A9 (Biosensors International, Singapore), AP23572 (Ariad Pharmaceuticals), γ-hiridun, clobetasol, pimecrolimus, imatinib mesylate, midostaurin, feno fibrate, prodrugs thereof, co-drugs thereof, and combinations thereof. Some other examples of the bioactive agent include siRNA and/or other oligoneucleotides that inhibit endothelial cell migration. Some further examples of the bioactive agent can also be lysophosphatidic acid (LPA) or sphingosine-1-phosphate (S1P). LPA is a "bioactive" phospholipid able to generate growth factor-like activities in a wide variety of normal and malignant cell types. LPA plays an important role in normal physiological processes such as wound healing, and in vascular tone, vascular integrity, or reproduction. As used herein, in some embodiments, the term "drug" and the term "bioactive agent" are used interchangeably.

The implantable device described herein can be formed on an implantable device such as a stent, which can be implanted in a patient to treat, prevent, mitigate, or reduce a vascular medical condition, or to provide a pro-healing effect. [0018] In some embodiments, the vascular medical condition or vascular condition is a coronary artery disease (CAD) and/or a peripheral vascular disease (PVD). Some examples of such vascular medical diseases are restenosis and/or atherosclerosis. Some other examples of these conditions include thrombosis, hemorrhage, vascular dissection or perforation, vascular aneurysm, vulnerable plaque, chronic total occlusion, claudication, anastomotic proliferation (for vein and artificial grafts), bile duct obstruction, urethral obstruction, tumor obstruction, or combinations of these.

### Definitions

Wherever applicable, the definitions to some terms used throughout the description of the present invention as provided below shall apply.
The terms "biologically degradable" (or "biodegradable"), "biologically erodable" (or "bioerodable"), "biologically absorbable" (or "bioabsorbable"), and "biologically resorbable" (or "bioresorbable"), in reference to polymers and coatings, are used interchangeably and refer to polymers and coatings that are capable of being completely or substantially completely degraded, dissolved, and/or eroded over time when exposed to physiological conditions and can be gradually resorbed, absorbed and/or eliminated by the body, or that can be degraded into fragments that can pass through the kidney membrane of an animal (e.g., a human), e.g., fragments having a molecular weight of about 40,000 Daltons (40 K Daltons) or less. The process of breaking down and eventual absorption and elimination of the polymer or coating can be caused by, e.g., hydrolysis, metabolic processes, oxidation, enzymatic processes, bulk or surface erosion, and the like. Conversely, a "biostable" polymer or coating refers to a polymer or coating that is not biodegradable.

Whenever the reference is made to "biologically degradable," "biologically erodable," "biologically absorbable," and "biologically resorbable" stent coatings or polymers forming such stent coatings, it is understood that after the process of degradation, erosion, absorption, and/or resorption has been completed or substantially completed, no coating or substantially little coating will remain on the stent. Whenever the terms "degradable," "biodegradable," or "biologically degradable" are used in this application, they are intended to broadly include biologically degradable, biologically erodable, biologically absorbable, and biologically resorbable polymers or coatings.

"Physiological conditions" refer to conditions to which an implant is exposed within the body of an animal (e.g., a human). Physiological conditions include, but are not limited to, "normal" body temperature for that species of animal (approximately 37°C for a human) and an aqueous environment of physiologic ionic strength, pH and enzymes. In some cases, the body temperature of a particular animal may be above or below what would be considered "normal" body temperature for that species of animal. For example, the body temperature of a human may be above or below approximately 37°C in certain cases. The scope of the present invention encompasses such cases where the physiological conditions (e.g., body temperature) of an animal are not considered "normal."

In the context of a blood-contacting implantable device, a "prohealing" drug or agent refers to a drug or agent that has the property that it promotes or enhances re-endothelialization of arterial lumen to promote healing of the vascular tissue.

As used herein, a "co-drug" is a drug that is administered concurrently or sequentially with another drug to achieve a particular pharmacological effect. The effect may be general or specific. The co-drug may exert an effect different from that of the other drug, or it may promote, enhance or potentiate the effect of the other drug.

As used herein, the term "prodrug" refers to an agent rendered less active by a chemical or biological moiety, which metabolizes into or undergoes *in vivo* hydrolysis to form a drug or an active ingredient thereof. The term "prodrug" can be used interchangeably with terms such as "proagent", "latentiated drugs", "bioreversible derivatives", and "congeners". N.J. Harper, Drug latentiation, Prog Drug Res., 4: 221-294 (1962); E.B. Roche, Design of Biopharmaceutical Properties through Prodrugs and Analogs, Washington, DC: American Pharmaceutical Association (1977); A.A. Sinkula and S.H. Yalkowsky, Rationale for design of biologically reversible drug derivatives: prodrugs, J. Pharm. Sci., 64: 181-210 (1975). Use of the term "prodrug" usually implies a covalent link between a drug and a chemical moiety, though some authors also use it to characterize some forms of salts of the active drug molecule. Although there is no strict universal definition of a prodrug itself, and the definition may vary from author to author, prodrugs can generally be defined as pharmacologically less active chemical derivatives that can be converted *in vivo,* enzymatically or nonenzymatically, to the active, or more active, drug molecules that exert a therapeutic, prophylactic or diagnostic effect. Sinkula and Yalkowsky, above; V.J. Stella et al., Prodrugs: Do they have advantages in clinical practice?, Drug, 29: 455-473 (1985).

The terms "polymer" and "polymeric" refer to compounds that are the product of a polymerization reaction. These terms are inclusive of homopolymers (i.e., polymers obtained by polymerizing one type of monomer by either chain or condensation polymers), copolymers (i.e., polymers obtained by polymerizing two or more different types of monomers by either chain or condensation polymers), condensation polymers (polymers made from condensation polymerization, terpolymers, etc., including random (by either chain or condensation polymers), alternating (by either chain or condensation polymers), block (by either chain or condensation polymers), graft, dendritic, crosslinked and any other variations thereof.

As used herein, the term "implantable" refers to the attribute of being implantable in a mammal (e.g., a human being or patient) that meets the mechanical, physical, chemical, biological, and pharmacological requirements of a device provided by laws and regulations of a governmental agency (e.g., the U.S. FDA) such that the device is safe and effective for use as indicated by the device. As used herein, an "implantable device" may be any suitable substrate that can be implanted in a human or non-human animal. Examples of implantable devices include, but are not limited to, self-expandable stents, balloon-expandable stents, coronary stents, peripheral stents, stent-grafts, catheters, other expandable tubular devices for various bodily lumen or orifices, grafts, vascular grafts, arterio-venous grafts, by-pass grafts, pacemakers and defibrillators, leads and electrodes for the preceding, artificial heart valves, anastomotic clips, arterial closure devices, patent foramen ovale closure devices, cerebrospinal fluid shunts, and particles (e.g., drug-eluting particles, microparticles and nanoparticles). The stents may be intended for any vessel in the body, including neurological, carotid, vein graft, coronary, aortic, renal, iliac, femoral, popliteal vasculature, and urethral passages. An implantable device can be designed for the localized delivery of a therapeutic agent. A medicated implantable device may be constructed in part, e.g., by coating the device with a coating material containing a therapeutic agent. The body of the device may also contain a therapeutic agent.

An implantable device can be fabricated with a coating containing partially or completely a biodegradable/bioabsorbable/ bioerodable polymer, a biostable polymer, or a combination thereof. An implantable device itself can also be fabricated partially or completely from a biodegradable/bioabsorbable/ bioerodable polymer, a biostable polymer, or a combination thereof.

As used herein, a material that is described as a layer or a film (e.g., a coating) "disposed over" an indicated substrate (e.g., an implantable device) refers to, e.g., a coating of the material deposited directly or indirectly over at least a portion of the surface of the substrate. Direct depositing means that the coating is applied directly to the exposed surface of the substrate. Indirect depositing means that the coating is applied to an intervening layer that has been deposited directly or indirectly over the substrate. In some embodiments, the term a "layer" or a "film" excludes a film or a layer formed on a non-implantable device.

In the context of a stent, "delivery" refers to introducing and transporting the stent through a bodily lumen to a region, such as a lesion, in a vessel that requires treatment. "Deployment" corresponds to the expanding of the stent within the lumen at the treatment region. Delivery and deployment of a stent are accomplished by positioning the stent about one end of a catheter, inserting the end of the catheter through the skin into a bodily lumen, advancing the catheter in the bodily lumen to a desired treatment location, expanding the stent at the treatment location, and removing the catheter from the lumen.

As used herein, the term "amorphous" refers to having a crystallinity less than 50% in a terpolymer. In some embodiments, the term "amorphous" can refer to having a crystallinity less than about 40%, less than about 30%, less than about 20%, less than about 10%, less than about 5%, less than about 1%, less than about 0.5%, or less than about 0.1% in a terpolymer.

### Random terpolymer

The terpolymer described herein can have different contents of the lactide (A), glycolide (B), and a third, low T_{g} monomer (C). The terpolymer can be expressed in this general formula AₓB_{y}C_{z}, wherein x, y and z are ratios of A, B, and C, respectively. Within the terpolymer, monomers A, B, and C can have any sequence of arrangement, for example, ABC, BAC, CBA, ACB, ABAC, ABBC, BABC, BAAC, BACC, CBCA, CBBA, CBAA, ABACA, ABACB, ABACC, BABCA, BABCB, BABCC, etc. As outlined in some embodiments, a sequence of monomers or units can have more than one units of a monomer, which are described in more detail below.

Terpolymers with different contents of these three monomers have different properties with regard to, e.g., rate of degradation, mechanical properties, drug permeability, water permeability, and drug release rate, depending on a particular composition of the monomers in the terpolymer.

In some embodiments, the terpolymer can have a T_{g} below about 60 °C. This terpolymer can have units derived from D-lactide, L-lactide, or D,L-lactide from about 10% to about 80% by weight. Monomers such as D-lactide, L-lactide, glycolide, and dioxanone can crystallalize if present in high concentration in a polymer. However, crystallization of units from any of these monomers can be minimized or prevented if concentration of each is below 80% by weight in the polymer. Therefore, the composition of a terpolymer described herein shall include units of D-lactide or L-lactide at about 10-80% by weight, units of glycolide at about 5-80% by weight and units from the third, low T_{g} monomer at about 5-60% by weight. The terpolymer can have a weight-average molecular weight (M_{w}) of about 10K Daltons or above, preferrably from about 20K Daltons to about 600K Daltons.

Ratios of units from the lactide, glycolide and the low T_{g} monomers can vary, forming a terpolymer having different properties, e.g., different degradation rates, different rates of release of a drug from a coating formed of the terpolymer, different drug permeability, different flexibility or mechanical properties. As noted above, generally, the glycolide provides an accelerated or enhanced degradation of the terpolymer, the lactide monomer provides mechanical strength to the terpolymer, and the third, low T_{g} monomer can enhance drug permeability, water permeability, and enhancing degradation rate of the polymer, imparting greater flexibility and elongation, and improving mechanical properties of a coating formed of the terpolymer.

In some embodiments, the ratio of the various monomers can vary along the chain of the terpolymer. In such a terpolymer, one point of the chain of polymer can be heavy with one monomer while another rpoint of the chain can be light with the same monomer, for example. If a monofunctional initiator is used, and if the selected monomers have highly different reactivity ratios, then a gradient of composition is generated as the monomers are consumed during the polymerization. In another methodology, such a terpolymer can be prepared by so-called gradient polymerization wherein during the polymerization a first or second monomer is progressively added to the reactor containing all, or a portion of, the first monomer. (Matyjaszewski K. and Davis T.P. eds. Handbook of Radical Polymerization, John Wiley & Sons, 2002, p. 789). Yet a third method is by introducing blocks of various ratios of the monomers into the chain of the terpolymer.

In some embodiments, the terpolymer described herein can be used to build one or more blocks in combination with other blocks such as poly(ethylene glycol) (PEG) or other blocks of biodegradable or biodurable polymers described below.

Randomness of the terpolymer described herein can be measured by randomness index. Generally, a perfectly alternating co-polymer would have a degree of randomness of 1. Conversely, in some embodiments, the terpolymer can include all the repeating units of the monomers in three blocks, the lactide block, the glycolide block, and the block of the third, low T_{g} monomer. Such a terpolymer would have a degree of randomness of 0. These are known as block copolymers. In some other embodiments, the terpolymer can have a degree of randomness ranging from above 0 to below 1, for example, about 0.01, about 0.02, about 0.05, about 0.1, about 0.2, about 0.25, about 0.3, about 0.35, about 0.4, about 0.45, about 0.5, about 0.55, about 0.6, about 0.65, about 0.7, about 0.75, about 0.8, about 0.85, about 0.9, about 0.95, or about 0.99. Generally, for a crystalline domain to develop, one usually needs a pentad (i.e. the same 5 repeat units or monomers in sequence). Therefore, in some embodiments, one factor to control the randomness of the terpolymer is to keep the repeat units or monomers in sequence in the terpolymer below 5, e.g., 1, 2, 3, or 4.

Randomness in a polymer can be readily determined by established techniques in the art. One such technique is NMR analysis ((see, e.g., J. Kasperczyk, Polymer, 37(2):201-203 (1996); Mangkorn Srisa-ard, et al., Polym Int., 50:891-896 (2001)).

Randomness of an amorphous terpolymer can be readily controlled or varied using techniques known in the art. For example, randomness in a batch reactor is controlled by polymerization temperature and type of solvent where the monomer reactivity ratios will change. For continuous reactors, it will also depend on monomer feed ratios and temperature. Secondarily, there is also a pressure effect on reactivity ratios. Monomers relative reactivity is also important, so you can control it by selecting monomers with similar or different reactivity.

As mentioned previously, one requisite attribute of the third monomer is that, if a homopolymer were to be formed of the third monomer, the homopolymer would have a T_{g} below about -20 °C. The third monomer can be any monomer that is capable of forming a terpolymer with lactide and glycolide. In some embodiments, the third low T_{g} monomer is a lactone, a carbonate, a thiocarbonate, an oxaketocycloalkane, or a thiooxaketocyclolakane. In some embodiments, the lactone, carbonate, thiocarbonate, oxaketocycloalkane, or thiooxaketocyclolakane can have hydrocarbyl or alkoxy substituent(s). In some embodiments, the substituent(s) can include hetero atom(s) such as a halo (F, Cl, Br or I) group(s). Some examples of substituents include, but are not limited to, methoxy, ethoxy, or a C1-C12 hydrocarbon group.

Some examples of the third monomer are given below in Table 1.

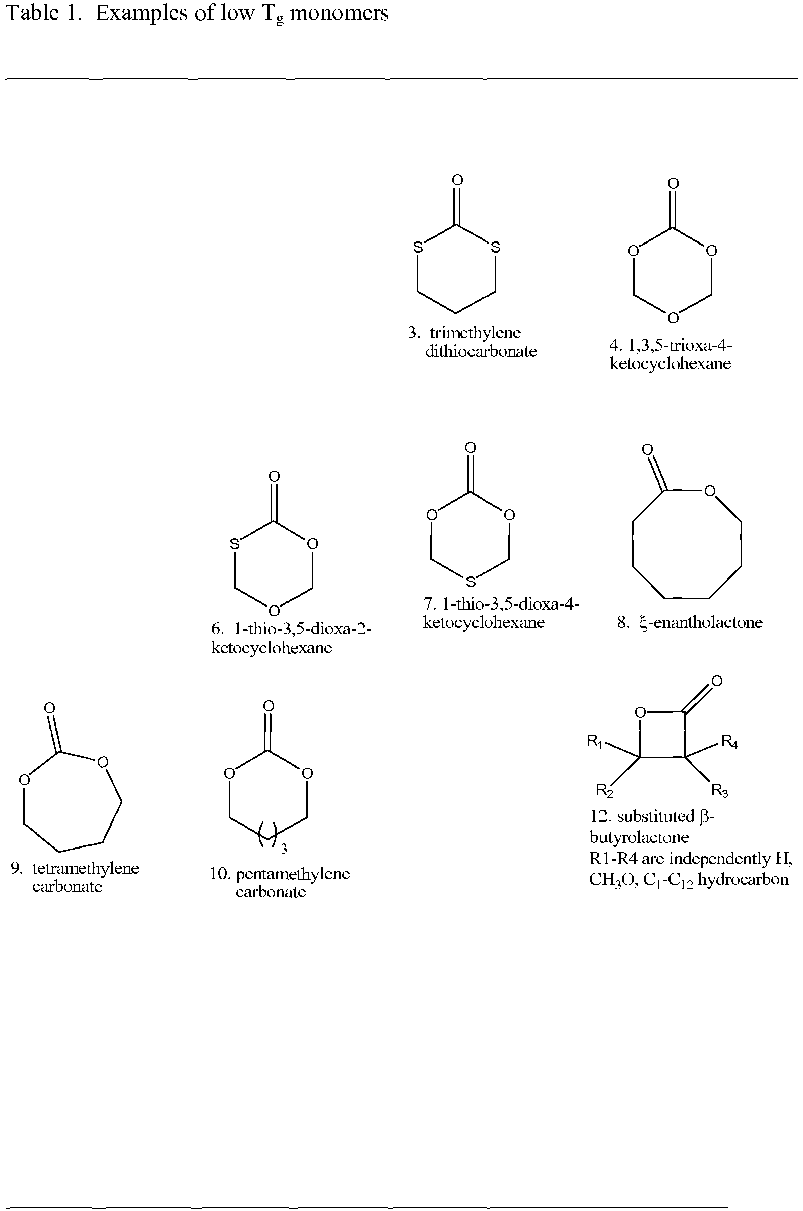

In some embodiments, monomers such as meso-lactide or thiolactones can be used to form a terpolymer with the third monomer. In these embodiments, the meso-lactide or thiolactones can be used with lactide or can replace lactide to form a terpolymer with the third, low T_{g} monomer.

Preparation of the terpolymer described herein can be readily accomplished by established methods of polymer synthesis. For example, a chosen composition of lactide, glycolide, and the third monomer with any of the various ratios described above can be subject to ring opening polymerization (ROP) to form a terpolymer. Polymer synthesis by ROP is a well-documented method of polymer synthesis and can be readily carried out by a person of ordinary skill in the art. Some other methods of polymer synthesis include, e.g., acid catalyzed polycondensation with removal of water. This would start with the monomers in hydroxyl-acid form, or as the cylic ester precursors. During the polymerization the water formed would be distilled off. Alternatively, room temperature polycondensations of the monomers in hydroxyl-acid form could be performed by using Mitsunobo conditions (DEAD/TPP) or by using dicyclohexylcarbodiimide with dimethylaminopyridine (DMAP) salt.

### Biologically Active Agents

In some embodiments, the implantable device described herein can optionally include at least one biologically active ("bioactive") agent. The at least one bioactive agent can include any substance capable of exerting a therapeutic, prophylactic or diagnostic effect for a patient.

Examples of suitable bioactive agents include, but are not limited to, synthetic inorganic and organic compounds, proteins and peptides, polysaccharides and other sugars, lipids, and DNA and RNA nucleic acid sequences having therapeutic, prophylactic or diagnostic activities. Nucleic acid sequences include genes, antisense molecules that bind to complementary DNA to inhibit transcription, and ribozymes. Some other examples of other bioactive agents include antibodies, receptor ligands, enzymes, adhesion peptides, blood clotting factors, inhibitors or clot dissolving agents such as streptokinase and tissue plasminogen activator, antigens for immunization, hormones and growth factors, oligonucleotides such as antisense oligonucleotides and ribozymes and retroviral vectors for use in gene therapy. The bioactive agents could be designed, e.g., to inhibit the activity of vascular smooth muscle cells. They could be directed at inhibiting abnormal or inappropriate migration and/or proliferation of smooth muscle cells to inhibit restenosis.

In certain embodiments, optionally in combination with one or more other embodiments described herein, the implantable device can include at least one biologically active agent selected from antiproliferative, antineoplastic, antimitotic, anti-inflammatory, antiplatelet, anticoagulant, antifibrin, antithrombin, antibiotic, antiallergic and antioxidant substances.

An antiproliferative agent can be a natural proteineous agent such as a cytotoxin or a synthetic molecule. Examples of antiproliferative substances include, but are not limited to, actinomycin D or derivatives and analogs thereof (manufactured by Sigma-Aldrich, or COSMEGEN available from Merck) (synonyms of actinomycin D include dactinomycin, actinomycin IV, actinomycin I₁, actinomycin X₁, and actinomycin C₁); all taxoids such as taxols, docetaxel, and paclitaxel and derivatives thereof; all olimus drugs such as macrolide antibiotics, rapamycin, everolimus, structural derivatives and functional analogues of rapamycin, structural derivatives and functional analogues of everolimus, FKBP-12 mediated mTOR inhibitors, biolimus, perfenidone, prodrugs thereof, co-drugs thereof, and combinations thereof. Examples of rapamycin derivatives include, but are not limited to, 40-*O*-(2-hydroxy)ethyl-rapamycin (trade name everolimus from Novartis), 40-*O*-(2-ethoxy)ethyl-rapamycin (biolimus), 40-*O*-(3-hydroxy)propyl-rapamycin, 40-*O*-[2-(2-hydroxy)ethoxy]ethyl-rapamycin, 40-*O*-tetrazole-rapamycin, 40-epi-(N1-tetrazolyl)-rapamycin (zotarolimus, manufactured by Abbott Labs.), Biolimus A9 (Biosensors International, Singapore), AP23572 (Ariad Pharmaceuticals), prodrugs thereof, co-drugs thereof, and combinations thereof.

An anti-inflammatory drug can be a steroidal anti-inflammatory drug, a nonsteroidal anti-inflammatory drug (NSAID), or a combination thereof. Examples of anti-inflammatory drugs include, but are not limited to, alclofenac, alclometasone dipropionate, algestone acetonide, alpha amylase, amcinafal, amcinafide, amfenac sodium, amiprilose hydrochloride, anakinra, anirolac, anitrazafen, apazone, balsalazide disodium, bendazac, benoxaprofen, benzydamine hydrochloride, bromelains, broperamole, budesonide, carprofen, cicloprofen, cintazone, cliprofen, clobetasol, clobetasol propionate, clobetasone butyrate, clopirac, cloticasone propionate, cormethasone acetate, cortodoxone, deflazacort, desonide, desoximetasone, dexamethasone, dexamethasone acetate, dexamethasone dipropionate, diclofenac potassium, diclofenac sodium, diflorasone diacetate, diflumidone sodium, diflunisal, difluprednate, diftalone, dimethyl sulfoxide, drocinonide, endrysone, enlimomab, enolicam sodium, epirizole, etodolac, etofenamate, felbinac, fenamole, fenbufen, fenclofenac, fenclorac, fendosal, fenpipalone, fentiazac, flazalone, fluazacort, flufenamic acid, flumizole, flunisolide acetate, flunixin, flunixin meglumine, fluocortin butyl, fluorometholone acetate, fluquazone, flurbiprofen, fluretofen, fluticasone propionate, furaprofen, furobufen, halcinonide, halobetasol propionate, halopredone acetate, ibufenac, ibuprofen, ibuprofen aluminum, ibuprofen piconol, ilonidap, indomethacin, indomethacin sodium, indoprofen, indoxole, intrazole, isoflupredone acetate, isoxepac, isoxicam, ketoprofen, lofemizole hydrochloride, lomoxicam, loteprednol etabonate, meclofenamate sodium, meclofenamic acid, meclorisone dibutyrate, mefenamic acid, mesalamine, meseclazone, methylprednisolone suleptanate, momiflumate, nabumetone, naproxen, naproxen sodium, naproxol, nimazone, olsalazine sodium, orgotein, orpanoxin, oxaprozin, oxyphenbutazone, paranyline hydrochloride, pentosan polysulfate sodium, phenbutazone sodium glycerate, pirfenidone, piroxicam, piroxicam cinnamate, piroxicam olamine, pirprofen, prednazate, prifelone, prodolic acid, proquazone, proxazole, proxazole citrate, rimexolone, romazarit, salcolex, salnacedin, salsalate, sanguinarium chloride, seclazone, sermetacin, sudoxicam, sulindac, suprofen, talmetacin, talniflumate, talosalate, tebufelone, tenidap, tenidap sodium, tenoxicam, tesicam, tesimide, tetrydamine, tiopinac, tixocortol pivalate, tolmetin, tolmetin sodium, triclonide, triflumidate, zidometacin, zomepirac sodium, aspirin (acetylsalicylic acid), salicylic acid, corticosteroids, glucocorticoids, tacrolimus, pimecorlimus, prodrugs thereof, co-drugs thereof, and combinations thereof.

Alternatively, the anti-inflammatory agent can be a biological inhibitor of pro-inflammatory signaling molecules. Anti-inflammatory biological agents include antibodies to such biological inflammatory signaling molecules.

In addition, the bioactive agents can be other than antiproliferative or anti-inflammatory agents. The bioactive agents can be any agent that is a therapeutic, prophylactic or diagnostic agent. In some embodiments, such agents can be used in combination with antiproliferative or anti-inflammatory agents. These bioactive agents can also have antiproliferative and/or anti-inflammmatory properties or can have other properties such as antineoplastic, antimitotic, cystostatic, antiplatelet, anticoagulant, antifibrin, antithrombin, antibiotic, antiallergic, and/or antioxidant properties.

Examples of antineoplastics and/or antimitotics include, but are not limited to, paclitaxel (e.g., TAXOL® available from Bristol-Myers Squibb), docetaxel (e.g., Taxotere® from Aventis), methotrexate, azathioprine, vincristine, vinblastine, fluorouracil, doxorubicin hydrochloride (e.g., Adriamycin® from Pfizer), and mitomycin (e.g., Mutamycin® from Bristol-Myers Squibb).

Examples of antiplatelet, anticoagulant, antifibrin, and antithrombin agents that can also have cytostatic or antiproliferative properties include, but are not limited to, sodium heparin, low molecular weight heparins, heparinoids, hirudin, argatroban, forskolin, vapiprost, prostacyclin and prostacyclin analogues, dextran, D-phe-pro-arg-chloromethylketone (synthetic antithrombin), dipyridamole, glycoprotein IIb/IIIa platelet membrane receptor antagonist antibody, recombinant hirudin, thrombin inhibitors such as ANGIOMAX (from Biogen), calcium channel blockers (e.g., nifedipine), colchicine, fibroblast growth factor (FGF) antagonists, fish oil (e.g., omega 3-fatty acid), histamine antagonists, lovastatin (a cholesterol-lowering drug that inhibits HMG-CoA reductase, brand name Mevacor® from Merck), monoclonal antibodies (e.g., those specific for platelet-derived growth factor (PDGF) receptors), nitroprusside, phosphodiesterase inhibitors, prostaglandin inhibitors, suramin, serotonin blockers, steroids, thioprotease inhibitors, triazolopyrimidine (a PDGF antagonist), nitric oxide or nitric oxide donors, super oxide dismutases, super oxide dismutase mimetics, 4-amino-2,2,6,6-tetramethylpiperidine-1-oxyl (4-amino-TEMPO), estradiol, anticancer agents, dietary supplements such as various vitamins, and a combination thereof.

Examples of cytostatic substances include, but are not limited to, angiopeptin, angiotensin converting enzyme inhibitors such as captopril (e.g., Capoten® and Capozide® from Bristol-Myers Squibb), cilazapril and lisinopril (e.g., Prinivil® and Prinzide® from Merck).

Examples of antiallergic agents include, but are not limited to, permirolast potassium. Examples of antioxidant substances include, but are not limited to, 4-amino-2,2,6,6-tetramethylpiperidine-1-oxyl (4-amino-TEMPO). Other bioactive agents include anti-infectives such as antiviral agents; analgesics and analgesic combinations; anorexics; antihelmintics; antiarthritics, antiasthmatic agents; anticonvulsants; antidepressants; antidiuretic agents; antidiarrheals; antihistamines; antimigrain preparations; antinauseants; antiparkinsonism drugs; antipruritics; antipsychotics; antipyretics; antispasmodics; anticholinergics; sympathomimetics; xanthine derivatives; cardiovascular preparations including calcium channel blockers and beta-blockers such as pindolol and antiarrhythmics; antihypertensives; diuretics; vasodilators including general coronary vasodilators; peripheral and cerebral vasodilators; central nervous system stimulants; cough and cold preparations, including decongestants; hypnotics; immunosuppressives; muscle relaxants; parasympatholytics; psychostimulants; sedatives; tranquilizers; naturally derived or genetically engineered lipoproteins; and restenoic reducing agents.

Other biologically active agents that can be used include alpha-interferon, genetically engineered epithelial cells, tacrolimus and dexamethasone.

A "prohealing" drug or agent, in the context of a blood-contacting implantable device, refers to a drug or agent that has the property that it promotes or enhances re-endothelialization of arterial lumen to promote healing of the vascular tissue. The portion(s) of an implantable device (e.g., a stent) containing a prohealing drug or agent can attract, bind, and eventually become encapsulated by endothelial cells (e.g., endothelial progenitor cells). The attraction, binding, and encapsulation of the cells will reduce or prevent the formation of emboli or thrombi due to the loss of the mechanical properties that could occur if the stent was insufficiently encapsulated. The enhanced re-endothelialization can promote the endothelialization at a rate faster than the loss of mechanical properties of the stent.

The prohealing drug or agent can be dispersed in the body of the bioabsorbable polymer substrate or scaffolding. The prohealing drug or agent can also be dispersed within a bioabsorbable polymer coating over a surface of an implantable device (e.g., a stent).

"Endothelial progenitor cells" refer to primitive cells made in the bone marrow that can enter the bloodstream and go to areas of blood vessel injury to help repair the damage. Endothelial progenitor cells circulate in adult human peripheral blood and are mobilized from bone marrow by cytokines, growth factors, and ischemic conditions. Vascular injury is repaired by both angiogenesis and vasculogenesis mechanisms. Circulating endothelial progenitor cells contribute to repair of injured blood vessels mainly via a vasculogenesis mechanism.

In some embodiments, the prohealing drug or agent can be an endothelial cell (EDC)-binding agent. In certain embodiments, the EDC-binding agent can be a protein, peptide or antibody, which can be, e.g., one of collagen type 1, a 23 peptide fragment known as single chain Fv fragment (scFv A5), a junction membrane protein vascular endothelial (VE)-cadherin, and combinations thereof. Collagen type 1, when bound to osteopontin, has been shown to promote adhesion of endothelial cells and modulate their viability by the down regulation of apoptotic pathways. S.M. Martin, et al, J. Biomed. Matter. Res., 70A:10-19 (2004). Endothelial cells can be selectively targeted (for the targeted delivery of immunoliposomes) using scFv A5. T. Volkel, et al., Biochimica et Biophysica Acts, 1663:158-166 (2004). Junction membrane protein vascular endothelial (VE)-cadherin has been shown to bind to endothelial cells and down regulate apoptosis of the endothelial cells. R. Spagnuolo, et al, Blood, 103:3005-3012 (2004).

In a particular embodiment, the EDC-binding agent can be the active fragment of osteopontin, (Asp-Val-Asp-Val-Pro-Asp-Gly-Asp-Ser-Leu-Ala-Try-Gly). Other EDC-binding agents include, but are not limited to, EPC (epithelial cell) antibodies, RGD peptide sequences, RGD mimetics, and combinations thereof.

In further embodiments, the prohealing drug or agent can be a substance or agent that attracts and binds endothelial progenitor cells. Representative substances or agents that attract and bind endothelial progenitor cells include antibodies such as CD-34, CD-133 and vegf type 2 receptor. An agent that attracts and binds endothelial progenitor cells can include a polymer having nitric oxide donor groups.

The foregoing biologically active agents are listed by way of example and are not meant to be limiting. Other biologically active agents that are currently available or that may be developed in the future are equally applicable.

In a more specific embodiment, optionally in combination with one or more other embodiments described herein, the implantable device of the invention comprises at least one biologically active agent selected from paclitaxel, docetaxel, estradiol, nitric oxide donors, super oxide dismutases, super oxide dismutase mimics, 4-amino-2,2,6,6-tetramethylpiperidine-1-oxyl (4-amino-TEMPO), tacrolimus, dexamethasone, dexamethasone acetate, rapamycin, rapamycin derivatives, 40-*O*-(2-hydroxy)ethyl-rapamycin (everolimus), 40-*O*-(2-ethoxy)ethyl-rapamycin (biolimus), 40-*O*-(3-hydroxy)propyl-rapamycin, 40-*O*-[2-(2-hydroxy)ethoxy]ethyl-rapamycin, 40-*O*-tetrazole-rapamycin, 40-epi-(N1-tetrazolyl)-rapamycin (zotarolimus), Biolimus A9 (Biosensors International, Singapore), AP23572 (Ariad Pharmaceuticals), pimecrolimus, imatinib mesylate, midostaurin, clobetasol, progenitor cell-capturing antibodies, prohealing drugs, prodrugs thereof, co-drugs thereof, and a combination thereof. In a particular embodiment, the bioactive agent is everolimus. In another specific embodiment, the bioactive agent is clobetasol.

An alternative class of drugs would be p-para-α-agonists for increased lipid transportation, examples include feno fibrate.

In some embodiments, optionally in combination with one or more other embodiments described herein, the at least one biologically active agent specifically cannot be one or more of any of the bioactive drugs or agents described herein.

### Coating construct

According to some embodiments of the invention, optionally in combination with one or more other embodiments described herein, a coating disposed over an implantable device (e.g., a stent) can include an amorphous terpolymer described herein in a layer according to any design of a coating. The coating can be a multi-layer structure that includes at least one reservoir layer, which is layer (2) described below, and can include any of the following (1), (3), (4) and (5) layers or combination thereof:
(1) a primer layer; (optional)
(2) a reservoir layer (also referred to "matrix layer" or "drug matrix"), which can be a drug-polymer layer including at least one polymer (drug-polymer layer) or, alternatively, a polymer-free drug layer;
(3) a release control layer (also referred to as a "rate-limiting layer") (optional);
(4) a topcoat layer; and/or (optional);
(5) a finishing coat layer. (optional).

In some embodiments, a coating of the invention can include two or more reservoir layers described above, each of which can include a bioactive agent described herein.

Each layer of a stent coating can be disposed over the implantable device (e.g., a stent) by dissolving the amorphous polymer, optionally with one or more other polymers, in a solvent, or a mixture of solvents, and disposing the resulting coating solution over the stent by spraying or immersing the stent in the solution. After the solution has been disposed over the stent, the coating is dried by allowing the solvent to evaporate. The process of drying can be accelerated if the drying is conducted at an elevated temperature. The complete stent coating can be optionally annealed at a temperature between about 40°C and about 150°C, e.g., 80°C, for a period of time between about 5 minutes and about 60 minutes, if desired, to allow for crystallization of the polymer coating, and/or to improve the thermodynamic stability of the coating.

To incorporate a bioactive agent (e.g., a drug) into the reservoir layer, the drug can be combined with the polymer solution that is disposed over the implantable device as described above. Alternatively, if it is desirable a polymer-free reservoir can be made. To fabricate a polymer-free reservoir, the drug can be dissolved in a suitable solvent or mixture of solvents, and the resulting drug solution can be disposed over the implantable device (e.g., stent) by spraying or immersing the stent in the drug-containing solution.

Instead of introducing a drug via a solution, the drug can be introduced as a colloid system, such as a suspension in an appropriate solvent phase. To make the suspension, the drug can be dispersed in the solvent phase using conventional techniques used in colloid chemistry. Depending on a variety of factors, e.g., the nature of the drug, those having ordinary skill in the art can select the solvent to form the solvent phase of the suspension, as well as the quantity of the drug to be dispersed in the solvent phase. Optionally, a surfactant can be added to stabilize the suspension. The suspension can be mixed with a polymer solution and the mixture can be disposed over the stent as described above. Alternatively, the drug suspension can be disposed over the stent without being mixed with the polymer solution.

The drug-polymer layer can be applied directly or indirectly over at least a portion of the stent surface to serve as a reservoir for at least one bioactive agent (e.g., drug) that is incorporated into the reservoir layer. The optional primer layer can be applied between the stent and the reservoir to improve the adhesion of the drug-polymer layer to the stent. The optional topcoat layer can be applied over at least a portion of the reservoir layer and serves as a rate-limiting membrane that helps to control the rate of release of the drug. In one embodiment, the topcoat layer can be essentially free from any bioactive agents or drugs. If the topcoat layer is used, the optional finishing coat layer can be applied over at least a portion of the topcoat layer for further control of the drug-release rate and for improving the biocompatibility of the coating. Without the topcoat layer, the finishing coat layer can be deposited directly on the reservoir layer.

Sterilization of a coated medical device generally involves a process for inactivation of micropathogens. Such processes are well known in the art. A few examples are e-beam, ETO sterilization, and irradiation. Most, if not all, of these processes can involve an elevated temperature. For example, ETO sterilization of a coated stent generally involves heating above 50°C at humidity levels reaching up to 100% for periods of a few hours up to 24 hours. A typical EtO cycle would have the temperature in the enclosed chamber to reach as high as above 50°C within the first 3-4 hours then and fluctuate between 40°C to 50°C for 17-18 hours while the humidity would reach the peak at 100% and maintain above 80% during the fluctuation time of the cycle.

The process of the release of a drug from a coating having both topcoat and finishing coat layers includes at least three steps. First, the drug is absorbed by the polymer of the topcoat layer at the drug-polymer layer/topcoat layer interface. Next, the drug diffuses through the topcoat layer using the void volume between the macromolecules of the topcoat layer polymer as pathways for migration. Next, the drug arrives at the topcoat layer/finishing layer interface. Finally, the drug diffuses through the finishing coat layer in a similar fashion, arrives at the outer surface of the finishing coat layer, and desorbs from the outer surface. At this point, the drug is released into the blood vessel or surrounding tissue. Consequently, a combination of the topcoat and finishing coat layers, if used, can serve as a rate-limiting barrier. The drug can be released by virtue of the degradation, dissolution, and/or erosion of the layer(s) forming the coating, or via migration of the drug through the amorphous polymeric layer(s) into a blood vessel or tissue.

In one embodiment, any or all of the layers of the stent coating can be made of an amorphous terpolymer described herein, optionally having the properties of being biologically degradable/erodable/absorbable/resorbable, non-degradable/biostable polymer, or a combination thereof. In another embodiment, the outermost layer of the coating can be limited to an amorphous terpolymer as defined above.

To illustrate in more detail, in a stent coating having all four layers described above (i.e., the primer, the reservoir layer, the topcoat layer and the finishing coat layer), the outermost layer is the finishing coat layer, which can be made of an amorphous terpolymer described herein and optionally having the properties of being biodegradable or, biostable, or being mixed with an amorphous terpolymer. The remaining layers (i.e., the primer, the reservoir layer and the topcoat layer) optionally having the properties of being biodegradable or, biostable, or being mixed with an amorphous terpolymer. The polymer(s) in a particular layer may be the same as or different than those in any of the other layers, as long as the layer on the outside of another bioabsorbable should preferally also be bioabsorbable and degrade at a similar or faster relative to the inner layer. As another illustration, the coating can include a single matrix layer comprising a polymer described herein and a drug.

If a finishing coat layer is not used, the topcoat layer can be the outermost layer and should be made of an amorphous terpolymer described herein and optionally having the properties of being biodegradable or, biostable, or being mixed with an amorphous terpolymer. In this case, the remaining layers (i.e., the primer and the reservoir layer) optionally can also be fabricated of an amorphous terpolymer described herein and optionally having the properties of being biodegradable or, biostable, or being mixed with an amorphous terpolymer The polymer(s) in a particular layer may be the same as or different than those in any of the other layers, as long as the outside of another bioabsorbable should preferably also be bioabsorbable and degrade at a similar or faster relative to the inner layer.

If neither a finishing coat layer nor a topcoat layer is used, the stent coating could have only two layers - the primer and the reservoir. In such a case, the reservoir is the outermost layer of the stent coating and should be made of an amorphous terpolymer described herein and optionally having the properties of being biodegradable or, biostable, or being mixed with an amorphous terpolymer. The primer optionally can also be fabricated of an amorphous terpolymer described herein and optionally one or more biodegradable polymer(s), biostable polymer(s), or a combination thereof. The two layers may be made from the same or different polymers, as long as the layer on the outside of another bioabsorbable should preferably also be bioabsorbable and degrade at a similar or faster relative to the inner layer.

Any layer of a coating can contain any amount of an amorphous terpolymer described herein and optionally having the properties of being biodegradable or, biostable, or being mixed with an amorphous terpolymer. Non-limiting examples of bioabsorbable polymers and biocompatible polymers include poly(N-vinyl pyrrolidone); polydioxanone; polyorthoesters; polyanhydrides; poly(glycolic acid); poly(glycolic acid-co-trimethylene carbonate); polyphosphoesters; polyphosphoester urethanes; poly(amino acids); poly(trimethylene carbonate); poly(iminocarbonates); co-poly(ether-esters); polyalkylene oxalates; polyphosphazenes; biomolecules, e.g., fibrin, fibrinogen, cellulose, cellophane, starch, collagen, hyaluronic acid, and derivatives thereof (e.g., cellulose acetate, cellulose butyrate, cellulose acetate butyrate, cellulose nitrate, cellulose propionate, cellulose ethers, and carboxymethyl cellulose), polyurethane,; polyesters, polycarbonates, polyurethanes, poly(L-lactic acid-co-caprolactone) (PLLA-CL), poly(D-lactic acid-co-caprolactone) (PDLA-CL), poly(DL-lactic acid-co-caprolactone) (PDLLA-CL), poly(D-lactic acid-glycolic acid (PDLA-GA), poly(L-lactic acid-glycolic acid (PLLA-GA), poly(DL-lactic acid-glycolic acid (PDLLA-GA), poly(D-lactic acid-co-glycolide-co-caprolactone) (PDLA-GA-CL), poly(L-lactic acid-co-glycolide-co-caprolactone) (PLLA-GA-CL), poly(DL-lactic acid-co-glycolide-co-caprolactone) (PDLLA-GA-CL), poly(L-lactic acid-co-caprolactone) (PLLA-CL), poly(D-lactic acid-co-caprolactone) (PDLA-CL), poly(DL-lactic acid-co-caprolactone) (PDLLA-CL), poly(glycolide-co-caprolactone) (PGA-CL), or any copolymers thereof.

Any layer of a stent coating can also contain any amount of a non-degradable polymer, or a blend of more than one such polymer as long as it is not mixed with a bioabsorbable polymer or any layer underneath the non-degradable layer comprise a bioabsorbable polymer. Non-limiting examples of non-degradable polymers include poly(methyl methacrylate), poly(ethyl methacrylate), poly(butyl methacrylate), poly(2-ethylhexyl methacrylate), poly(lauryl methacrylate), poly(2-hydroxylethyl methacrylate), poly(ethylene glycol (PEG) acrylate), poly(PEG methacrylate), methacrylate polymers containing 2-methacryloyloxyethylphosphorylcholine (MPC), PC 1036, and poly(n-vinyl pyrrolidone, poly(methacrylic acid), poly(acrylic acid), poly(hydroxypropyl methacrylate), poly(hydroxypropyl methacrylamide), methacrylate polymers containing 3-trimethylsilylpropyl methacrylate, and copolymers thereof.

A coating formed of the terpolymer described herein can degrade within about 1 month, 2 months, 3 months, 4 months, 6 months, 12 months, 18 months, or 24 months after implantation of a medical device comprising the coating. In some embodiments, the coating can completely degrade or fully absorb within 24 months after implantation of a medical device comprising the coating.

### Method of Fabricating implantable Device

Other embodiments of the invention, optionally in combination with one or more other embodiments described herein, are drawn to a method of fabricating an implantable device. In one embodiment, the method comprises forming the implantable device of a material containing an amorphous terpolymer described herein, optionally with one or more other biodegradable or biostable polymer or copolymers.

Under the method, a portion of the implantable device or the whole device itself can be formed of the material containing a biodegradable or biostable polymer or copolymer. The method can deposit a coating having a range of thickness over an implantable device. In certain embodiments, the method deposits over at least a portion of the implantable device a coating that has a thickness of ≤ about 30 micron, or ≤ about 20 micron, or ≤ about 10 micron, or ≤ about 5 micron.

In certain embodiments, the method is used to fabricate an implantable device selected from stents, grafts, stent-grafts, catheters, leads and electrodes, clips, shunts, closure devices, valves, and particles. In a specific embodiment, the method is used to fabricate a stent.

In some embodiments, to form an implantable device formed from a polymer, a polymer or copolymer optionally including at least one bioactive agent described herein can be formed into a polymer construct, such as a tube or sheet that can be rolled or bonded to form a construct such as a tube. An implantable device can then be fabricated from the construct. For example, a stent can be fabricated from a tube by laser machining a pattern into the tube. In another embodiment, a polymer construct can be formed from the polymeric material of the invention using an injection-molding apparatus.

Non-limiting examples of polymers, which may or may not be the amorphous terpolymers defined above, that can be used to fabricate an implantable device include poly(N-acetylglucosamine) (Chitin), Chitosan, poly(hydroxyvalerate), poly(lactide-co-glycolide), poly(hydroxybutyrate), poly(hydroxybutyrate-co-valerate), polyorthoester, polyanhydride, poly(L-lactic acid-co-caprolactone) (PLLA-CL), poly(D-lactic acid-co-caprolactone) (PDLA-CL), poly(DL-lactic acid-co-caprolactone) (PDLLA-CL), poly(D-lactic acid-glycolic acid (PDLA-GA), poly(L-lactic acid-glycolic acid (PLLA-GA), poly(DL-lactic acid-glycolic acid (PDLLA-GA), poly(D-lactic acid-co-glycolide-co-caprolactone) (PDLA-GA-CL), poly(L-lactic acid-co-glycolide-co-caprolactone) (PLLA-GA-CL), poly(DL-lactic acid-co-glycolide-co-caprolactone) (PDLLA-GA-CL), poly(L-lactic acid-co-caprolactone) (PLLA-CL), poly(D-lactic acid-co-caprolactone) (PDLA-CL), poly(DL-lactic acid-co-caprolactone) (PDLLA-CL), poly(glycolide-co-caprolactone) (PGA-CL), poly(thioesters), poly(trimethylene carbonate), polyethylene amide, polyethylene acrylate, poly(glycolic acid-co-trimethylene carbonate), co-poly(ether-esters) (e.g., PEO/PLA), polyphosphazenes, biomolecules (e.g., fibrin, fibrinogen, cellulose, starch, collagen and hyaluronic acid), polyurethanes, silicones, polyesters, polyolefins, polyisobutylene and ethylene-alphaolefin copolymers, acrylic polymers and copolymers other than polyacrylates, vinyl halide polymers and copolymers (e.g., polyvinyl chloride), polyvinyl ethers (e.g., polyvinyl methyl ether), polyvinylidene halides (e.g., polyvinylidene chloride), polyacrylonitrile, polyvinyl ketones, polyvinyl aromatics (e.g., polystyrene), polyvinyl esters (e.g., polyvinyl acetate), acrylonitrile-styrene copolymers, ABS resins, polyamides (e.g., Nylon 66 and polycaprolactam), polycarbonates, polyoxymethylenes, polyimides, polyethers, polyurethanes, rayon, rayon-triacetate, cellulose and derivates thereof (e.g., cellulose acetate, cellulose butyrate, cellulose acetate butyrate, cellophane, cellulose nitrate, cellulose propionate, cellulose ethers, and carboxymethyl cellulose), and copolymers thereof.

Additional representative examples of polymers that may be suited for fabricating an implantable device include ethylene vinyl alcohol copolymer (commonly known by the generic name EVOH or by the trade name EVAL), poly(butyl methacrylate), poly(vinylidene fluoride-co-hexafluoropropylene) (e.g., SOLEF 21508, available from Solvay Solexis PVDF of Thorofare, New Jersey), polyvinylidene fluoride (otherwise known as KYNAR, available from ATOFINA Chemicals of Philadelphia, Pennsylvania), poly(tetrafluoroethylene-co- hexafluoropropylene-co-vinylidene fluoride), ethylene-vinyl acetate copolymers, and polyethylene glycol.

### Method of Treating or Preventing Disorders

An implantable device according to the present invention can be used to treat, prevent or diagnose various conditions or disorders. Examples of such conditions or disorders include, but are not limited to, atherosclerosis, thrombosis, restenosis, hemorrhage, vascular dissection, vascular perforation, vascular aneurysm, vulnerable plaque, chronic total occlusion, patent foramen ovale, claudication, anastomotic proliferation of vein and artificial grafts, arteriovenous anastamoses, bile duct obstruction, urethral obstruction and tumor obstruction. A portion of the implantable device or the whole device itself can be formed of the material, as described herein. For example, the material can be a coating disposed over at least a portion of the device.

In certain embodiments, optionally in combination with one or more other embodiments described herein, the inventive method treats, prevents or diagnoses a condition or disorder selected from atherosclerosis, thrombosis, restenosis, hemorrhage, vascular dissection, vascular perforation, vascular aneurysm, vulnerable plaque, chronic total occlusion, patent foramen ovale, claudication, anastomotic proliferation of vein and artificial grafts, arteriovenous anastamoses, bile duct obstruction, urethral obstruction and tumor obstruction. In a particular embodiment, the condition or disorder is atherosclerosis, thrombosis, restenosis or vulnerable plaque.

In one embodiment of the method, optionally in combination with one or more other embodiments described herein, the implantable device is formed of a material or includes a coating containing at least one biologically active agent selected from paclitaxel, docetaxel, estradiol, nitric oxide donors, super oxide dismutases, super oxide dismutase mimics, 4-amino-2,2,6,6-tetramethylpiperidine-1-oxyl (4-amino-TEMPO), tacrolimus, dexamethasone, dexamethasone acetate, rapamycin, rapamycin derivatives, 40-*O*-(2-hydroxy)ethyl-rapamycin (everolimus), 40-*O*-(2-ethoxy)ethyl-rapamycin (biolimus), 40-*O*-(3-hydroxy)propyl-rapamycin, 40-*O*-[2-(2-hydroxy)ethoxy]ethyl-rapamycin, 40-*O*-tetrazole-rapamycin, 40-epi-(N1-tetrazolyl)-rapamycin (zotarolimus), Biolimus A9 (Biosensors International, Singapore), AP23572 (Ariad Pharmaceuticals), pimecrolimus, imatinib mesylate, midostaurin, clobetasol, progenitor cell-capturing antibodies, prohealing drugs, fenofibrate, prodrugs thereof, co-drugs thereof, and a combination thereof.

In certain embodiments, optionally in combination with one or more other embodiments described herein, the implantable device used in the method is selected from stents, grafts, stent-grafts, catheters, leads and electrodes, clips, shunts, closure devices, valves, and particles. In a specific embodiment, the implantable device is a stent.

## Claims

1. An amorphous random terpolymer for controlling the release of a bioactive agent, wherein the terpolymer comprises units derived from a lactide, glycolide, and a low glass transition temperature (Tg) monomer,
wherein the lactide is selected from D-lactide, L-lactide, D,L-lactide, or meso-lactide and has a ratio of 5-80% by weight of the total monomers forming the terpolymer,
wherein the glycolide has a ratio of 5-80% by weight of the total monomers forming the terpolymer,
wherein the low T_{g} monomer is capable of forming a homopolymer having a T_{g} of -20 °C or below,
wherein the low T_{g} monomer is selected from: 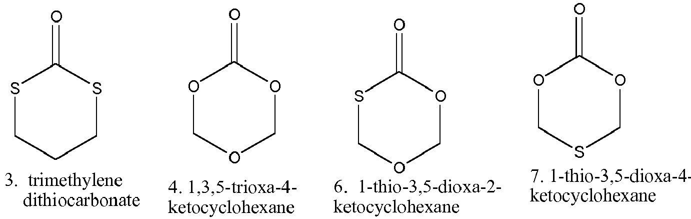 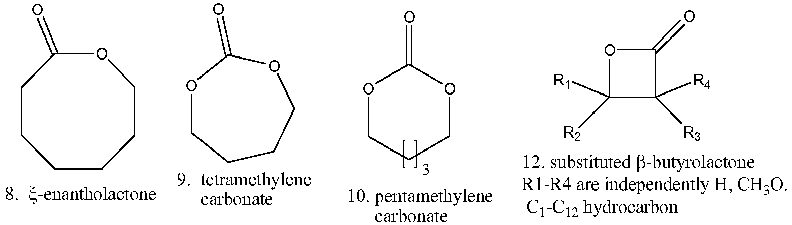 wherein the terpolymer has a T_{g} of 60°C or below and a weight average molecular weight (Mw) from 20K Daltons to 600K Daltons;
wherein the terpolymer has a crystallinity less than 50%; and
wherein the number of the repeat units or monomers in sequence in the terpolymer is below 5.

2. The terpolymer of claim 1, wherein the amorphous terpolymer has a degree of randomness ranges from above 0.5 to 1.

3. The terpolymer of claim 1, wherein the low T_{g} monomer has a ratio of 5-60% by weight of the total monomers forming the terpolymer.

4. A method of fabricating an implantable medical device, comprising forming a coating on the implantable device, the coating comprising an amorphous random terpolymer as defined in any of the claims 1-3.

5. The method of claim 4, further comprising the addition of one or more bioactive agent.

6. The method of claim 5, wherein the bioactive agent is selected from paclitaxel, docetaxel, estradiol, 17-beta-estradiol, nitric oxide donors, super oxide dismutases, super oxide dismutases mimics, 4-amino-2,2,6,6-tetramethylpiperidine-1-oxyl (4-amino-TEMPO), biolimus, tacrolimus, dexamethasone, dexamethasone acetate, rapamycin, rapamycin derivatives, 40-O-(2-hydroxy)ethyl-rapamycin (everolimus), 40-O-(3-hydroxy)propyl-rapamycin, 40-O-[2-(2-hydroxy)ethoxy]ethyl-rapamycin, and 40-O-tetrazole-rapamycin, 40-epi-(N1-tetrazolyl)-rapamycin (ABT-578), zotarolimus, 42-O-(2-ethoxylethyl)rapamycin, γ-hiridun, clobetasol, pimecrolimus, imatinib mesylate, midostaurin, feno fibrate, and combinations thereof.

7. An implantable device, comprising a coating that comprises an amorphous random terpolymer according to claim 1, wherein the coating is deposited on the implantable device and comprises at least one reservoir layer, wherein the implantable device further comprises one or more bioactive agents.

8. The implantable device of claim 7, wherein the amorphous terpolymer has a degree of randomness from above 0.5 to 1.

9. The implantable device of claim 7, wherein the low T_{g} monomer has a ratio of 5-60% by weight of the total monomers forming the terpolymer.

10. The implantable device of claim 9, wherein the bioactive agent is selected from paclitaxel, docetaxel, estradiol, 17-beta-estradiol, nitric oxide donors, super oxide dismutases, super oxide dismutases mimics, 4-amino-2,2,6,6-tetramethylpiperidine-1-oxyl (4-amino-TEMPO), biolimus, tacrolimus, dexamethasone, dexamethasone acetate, rapamycin, rapamycin derivatives, 40-O-(2-hydroxy)ethyl-rapamycin (everolimus), 40-O-(3-hydroxy)propyl-rapamycin, 40-O-[2-(2-hydroxy)ethoxy]ethyl-rapamycin, and 40-O-tetrazole-rapamycin, 40-epi-(N1-tetrazolyl)-rapamycin (ABT-578), zotarolimus 42-O-(2-ethoxylethyl)-rapamycin, γ-hiridun, clobetasol, pimecrolimus, imatinib mesylate, midostaurin, feno fibrate, and combinations thereof.

11. The implantable device of claim 7, which is a stent.

12. The implantable device of claim 7, which is a bioabsorbable stent.

13. The implantable device of claim 10, which is a bioabsorbable stent.

14. An implantable device as defined in claim 7, for use in the treatment, prevention, or amelioration of a vascular medical condition, wherein the vascular medical condition is selected from restenosis, atherosclerosis, thrombosis, hemorrhage, vascular dissection or perforation, vascular aneurysm, vulnerable plaque, chronic total occlusion, claudication, anastomotic proliferation (for vein and artificial grafts), bile duct obstruction, urethral obstruction, tumor obstruction, or combinations of these.

15. The implantable device as defined in claim 7, which further comprises any of a primer layer, a release control layer, a topcoat layer, a finish coat layer, or a combination thereof.

16. The implantable device of claim 7, wherein the reservoir layer comprises one or more bioactive agent.

17. The implantable device of claim 16, wherein the one or more bioactive agent is selected from paclitaxel, docetaxel, estradiol, 17-beta-estradiol, nitric oxide donors, super oxide dismutases, super oxide dismutases mimics, 4-amino-2,2,6,6-tetramethylpiperidine-1-oxyl (4-amino-TEMPO), biolimus, tacrolimus, dexamethasone, dexamethasone acetate, rapamycin, rapamycin derivatives, 40-O-(2-hydroxy)ethyrrapamycin (everolimus), 40-O-(3-hydroxy)propyl-rapamycin, 40-O-[2-(2-hydroxy)ethoxy]ethyl-rapamycin, and 40-O-tetrazole-rapamycin, 40-epi-(N1-tetrazolyl)-rapamycin (ABT-578), zotarolimus, 42-O-(2-ethoxylethyl)rapamycin, γ-hiridun, clobetasol, pimecrolimus, imatinib mesylate, midostaurin, feno fibrate, and combinations thereof.

18. A coating comprising an amorphous random terpolymer as defined in claim 1, and one or more bioactive agents.

## Patentansprüche

1. Ein amorphes statistisches Terpolymer zur Kontrolle der Freisetzung von einem Wirkstoff, wobei das Terpolymer Einheiten umfasst, die von einem Lactid, Glycolid und einem Monomer niedriger Glasübergangstemperatur (T_{g}) abgeleitet sind,
wobei das Lactid aus D-Lactid, L-Lactid, D,L-Lactid, oder Meso-Lactid ausgewählt ist und ein Verhältnis von 5-80 Gew.-% bezogen auf die Gesamtheit der das Terpolymer bildenden Monomere hat,
wobei das Glycolid ein Verhältnis von 5-80 Gew.-% bezogen auf die Gesamtheit der das Terpolymer bildenden Monomere hat,
wobei das Monomer niedriger T_{g} ein Homopolymer bilden kann, das eine T_{g} von -20 °C oder geringer hat,
wobei das Monomer niedriger T_{g} ausgewählt ist aus: 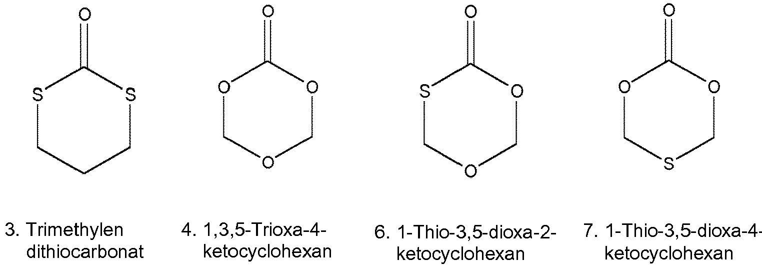 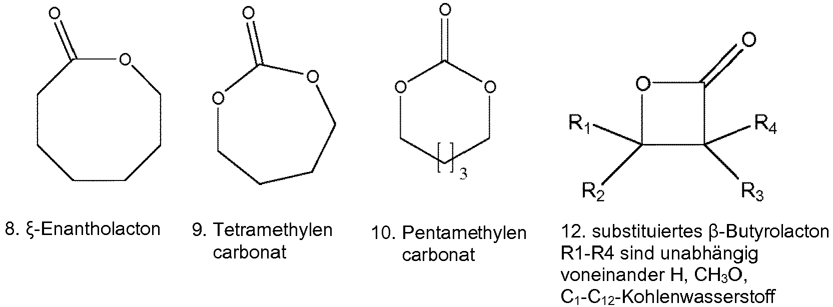 wobei das Terpolymer eine T_{g} von 60°C oder geringer hat und ein Gewichtsmittel des Molekulargewichts (Mw) von 20K Daltons bis 600K Daltons hat;
wobei das Terpolymer eine Kristallinität von weniger als 50% hat; und
wobei die Anzahl der Wiederholungseinheiten oder Monomere in Reihenfolge im Terpolymer kleiner als 5 ist.

2. Das Terpolymer des Anspruchs 1, wobei das amorphe Terpolymer eine Zufälligkeitsgrad von oberhalb 0,5 bis 1 hat.

3. Das Terpolymer des Anspruchs 1, wobei das Monomer geringer T_{g} ein Verhältnis von 5-60 Gew.-% bezogen auf die Gesamtheit der das Terpolymer bildenden Monomere hat.

4. Ein Verfahren zur Herstellung eines implantierbaren medizinischen Geräts, umfassend die Bildung von einer Beschichtung auf dem implantierbaren Gerät, wobei die Beschichtung ein amorphes statistisches Terpolymer wie in einem der Ansprüche 1-3 definiert umfasst.

5. Das Verfahren des Anspruchs 4, weiterhin umfassend die Zugabe von einem oder mehreren Wirkstoffen.

6. Das Verfahren des Anspruchs 5, wobei der Wirkstoff ausgewählt von Paclitaxel, Docetaxel, Estradiol, 17-beta-Estradiol, NO-Donatoren, Superoxid-Dismutasen, Superoxiddismutase-Mimetika, 4-Amino-2,2,6,6- tetramethylpiperidin-1-oxyl (4-Amino-TEMPO), Biolimus, Tacrolimus, Dexamethason, Dexamethasonacetat, Rapamycin, Derivaten von Rapamycin, 40-O-(2-Hydroxy)ethyl-rapamycin (Everolimus), 40-O-(3-Hydroxy)propyl-rapamycin, 40-O-[2-(2-Hydroxy)ethoxy]ethyl-rapamycin, und 40-O-Tetrazol-rapamycin, 40-Epi-(N1-tetrazolyl)-rapamycin (ABT-578), Zotarolimus, 42-O-(2-Ethoxylethyl)rapamycin, γ-Hiridun, Clobetasol, Pimecrolimus, Imatinibmesylat, Midostaurin, Fenofibrat, und Kombinationen davon ist.

7. Ein implantierbares Gerät umfassend eine Beschichtung, welche ein amorphes statistisches Terpolymer nach Anspruch 1 umfasst, wobei die Beschichtung auf dem implantierbaren Gerät aufgetragen wird und mindestens eine Reservoirschicht umfasst, wobei das implantierbare Gerät weiterhin einen oder mehrere Wirkstoffe umfasst.

8. Das implantierbare Gerät des Anspruchs 7, wobei das amorphe Terpolymer eine Zufälligkeitsgrad von oberhalb 0,5 bis 1 hat.

9. Das implantierbare Gerät des Anspruchs 7, wobei das Monomer geringer T_{g} ein Verhältnis von 5-60 Gew.% bezogen auf die Gesamtheit der das Terpolymer bildenden Monomere hat.

10. Das implantierbare Gerät des Anspruchs 9, wobei der Wirkstoff ausgewählt von Paclitaxel, Docetaxel, Estradiol, 17-beta-Estradiol, NO-Donatoren, Superoxid-Dismutasen, Superoxiddismutase-Mimetika, 4-Amino-2,2,6,6- tetramethylpiperidin-1-oxyl (4-Amino-TEMPO), Biolimus, Tacrolimus, Dexamethason, Dexamethasonacetat, Rapamycin, Derivaten von Rapamycin, 40-O-(2-Hydroxy)ethyl-rapamycin (Everolimus), 40-O-(3-Hydroxy)propyl-rapamycin, 40-O-[2-(2-Hydroxy)ethoxy]ethyl-rapamycin, und 40-O-Tetrazol-rapamycin, 40-Epi-(N1-tetrazolyl)-rapamycin (ABT-578), Zotarolimus, 42-O-(2-Ethoxylethyl)rapamycin, γ-Hiridun, Clobetasol, Pimecrolimus, Imatinibmesylat, Midostaurin, Fenofibrat, und Kombinationen davon ist.

11. Das implantierbare Gerät des Anspruchs 7, welches ein Stent ist.

12. Das implantierbare Gerät des Anspruchs 7, welches ein bioresorbierbarer Stent ist.

13. Das implantierbare Gerät des Anspruchs 10, welches ein bioresorbierbarer Stent ist.

14. Ein implantierbares Gerät wie in Anspruch 7 definiert, zur Verwendung bei der Behandlung, Prävention oder Linderung von einer medizinischen Gefäßerkrankung, wobei die medizinische Gefäßerkrankung ausgewählt von Restenose, Atherosclerose, Thrombose, Hämorrhagien, Gefäßdisektion oder Gefäßperforation, Gefäßaneurysma, vulnerabler Plaque, chronischem Totalverschluss, Claudicatio, anastomotischer Proliferation (für Venen- und künstliche Transplantate), Gallengänge-Obstruktion, Ureterobstruction, Tumorobstruktion, der Kombinationen davon ist.

15. Das implantierbare Gerät wie in Anspruch 7 definiert, welches weiterhin eine von einer Grundierschicht, einer Freisetzungskontrolleschicht, einer Oberschicht, einer Deckschicht, oder einer Kombination davon umfasst.

16. Das implantierbare Gerät des Anspruchs 7, wobei die Reservoirschicht einen oder mehrere Wirkstoffe umfasst.

17. Das implantierbare Gerät des Anspruchs 16, wobei der eine oder die mehreren Wirkstoffe ausgewählt von Paclitaxel, Docetaxel, Estradiol, 17-beta-Estradiol, NO-Donatoren, Superoxid-Dismutasen, Superoxiddismutase-Mimetika, 4-Amino-2,2,6,6-tetramethylpiperidin-1-oxyl (4-Amino-TEMPO), Biolimus, Tacrolimus, Dexamethason, Dexamethasonacetat, Rapamycin, Derivaten von Rapamycin, 40-O-(2-Hydroxy)ethyl-rapamycin (Everolimus), 40-O-(3-Hydroxy)propyl-rapamycin, 40-O-[2-(2-Hydroxy)ethoxy]ethyl-rapamycin, und 40-O-Tetrazol-rapamycin, 40-Epi-(N1-tetrazolyl)-rapamycin (ABT-578), Zotarolimus, 42-O-(2-Ethoxylethyl)rapamycin, γ-Hiridun, Clobetasol, Pimecrolimus, Imatinibmesylat, Midostaurin, Fenofibrat, und Kombinationen davon sind.

18. Eine Beschichtung umfassend ein amorphes statistisches Terpolymer wie in Anspruch 1 definiert, und einen oder mehrere Wirkstoffe.

## Revendications

1. Un terpolymère statistique amorphe pour contrôler la libération d'un agent bioactif, dans lequel le terpolymère comprend des unités dérivées d'un lactide, un glycolide, et un monomère à basse température de transition vitreuse (T_{g}),
dans lequel le lactide est choisi de D-lactid, L-lactid, D,L-lactide, ou mésolactide et a un rapport de 5-80% en poids des monomères totaux formant le terpolymère,
dans lequel le glycolide a un rapport de 5-80% en poids des monomères totaux formant le terpolymère,
dans lequel le monomère à basse T_{g} est capable de former un homopolymère ayant une T_{g} de -20 °C ou inférieure,
dans lequel le monomère à basse T_{g} est choisi parmi: 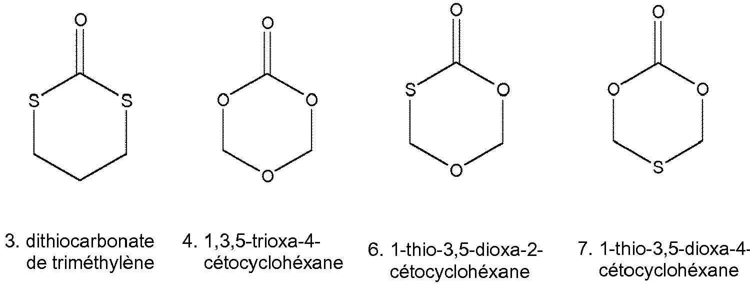 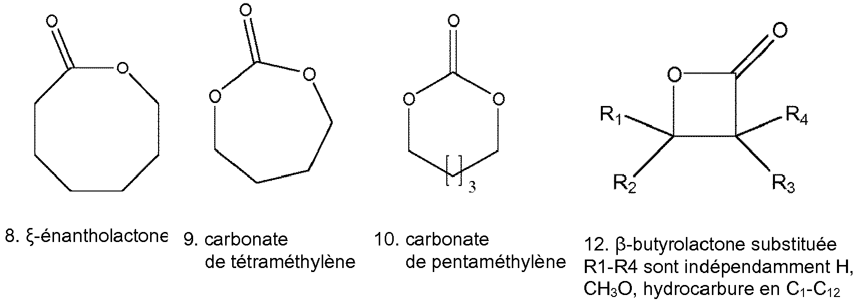 dans lequel le terpolymère a une T_{g} de 60 °C ou inférieure et une masse moléculaire moyenne en poids (Mw) allant de 20K Daltons à 600K Daltons;
dans lequel le terpolymère a une cristallinité inférieure à 50%; et
dans lequel le nombre d'unités répétitives ou monomères en séquence dans le terpolymère est inférieur à 5.

2. Le terpolymère de la revendication 1, dans lequel le terpolymère amorphe a un degré d'aléa allant de plus de 0,5 à 1.

3. Le terpolymère de la revendication 1, dans lequel le monomère à basse T_{g} a un rapport de 5-60% en poids des monomères totaux formant le terpolymère,

4. Un procédé de fabrication d'un dispositif médical implantable, comprenant la formation d'un revêtement sur le dispositif implantable, comprenant le revêtement un terpolymère statistique amorphe tel que défini dans l'une quelconque des revendications 1-3.

5. Le procédé de la revendication 4, comprenant en outre l'addition d'un ou de plusieurs agents bioactifs.

6. Le procédé de la revendication 5, dans lequel l'agent bioactif est choisi du paclitaxel, du docétaxel, de l'estradiol, du 17-béta-estradiol, des donneurs d'oxyde nitrique, des superoxyde dismutases, des mimétiques de superoxyde dismutases, du 4-amino-2,2,6,6- tetraméthylpipéridine-1-oxyle (4-amino-TEMPO), du biolimus, du tacrolimus, de la déxaméthasone, de l'acétate de déxaméthasone, de la rapamycine, des dérivés de la rapamycine, de la 40-O-(2-hydroxy)éthyl-rapamycine (évérolimus), de la 40-O-(3-hydroxy)propyl-rapamycine, de la 40-O-[2-(2-hydroxy)éthoxy]éthyl-rapamycine, et de la 40-O-tétrazole-rapamycine, de la 40-epi-(N1-tétrazolyl)-rapamycine (ABT-578), du zotarolimus, de la 42-O-(2- éthoxyléthyl)rapamycine, du y-hiridun, du clobétasol, du pimécrolimus, du mésylate d'imatinib, de la midostaurine, du fenofibrate, et des combinaisons de ceux-ci.

7. Un dispositif implantable, comprenant une couche qui comprend un terpolymère statistique amorphe selon la revendication 1, dans lequel le revêtement est déposé sur le dispositif implantable et comprend au moins une couche de réservoir, dans lequel le dispositif implantable comprend en outre un ou plusieurs agents bioactifs.

8. Le dispositif implantable de la revendication 7, dans lequel le terpolymère amorphe a un degré d'aléa allant de plus de 0,5 à 1.

9. Le dispositif implantable de la revendication 7, dans lequel le monomère à basse T_{g} a un rapport de 5-60% en poids des monomères totaux formant le terpolymère,

10. Le dispositif implantable de la revendication 9, dans lequel l'agent bioactif est choisi du paclitaxel, du docétaxel, de l'estradiol, du 17-béta-estradiol, des donneurs d'oxyde nitrique, des superoxyde dismutases, des mimétiques de superoxyde dismutases, du 4-amino-2,2,6,6- tetraméthylpipéridine-1-oxyle (4-amino-TEMPO), du biolimus, du tacrolimus, de la déxaméthasone, de l'acétate de déxaméthasone, de la rapamycine, des dérivés de la rapamycine, de la 40-O-(2-hydroxy)éthyl-rapamycine (évérolimus), de la 40-O-(3-hydroxy)propyl-rapamycine, de la 40-O-[2-(2-hydroxy)éthoxy]éthyl-rapamycine, et de la 40-O-tétrazole-rapamycine, de la 40-epi-(N1-tétrazolyl)-rapamycine (ABT-578), du zotarolimus, de la 42-O-(2-éthoxyléthyl)rapamycine, du y-hiridun, du clobétasol, du pimécrolimus, du mésylate d'imatinib, de la midostaurine, du fenofibrate, et des combinaisons de ceux-ci.

11. Le dispositif implantable de la revendication 7, qui est un stent.

12. Le dispositif implantable de la revendication 7, qui est un stent bioabsorbable.

13. Le dispositif implantable de la revendication 10, qui est un stent bioabsorbable.

14. Un dispositif implantable tel que défini dans la revendication 7, pour l'utilisation dans le traitement, prévention, ou amélioration d'une condition médicale vasculaire, dans lequel la condition médicale vasculaire est choisie de la resténose, l'athérosclerose, la thrombose, les hémorragies, la dissection ou la perforation vasculaire, l'anéurysme vasculaire, la plaque vulnérable, l'occlusion totale chronique, la claudication, la prolifération anastomotique (pour des implants de veines et des implants artificiels), l'obstruction des voies biliaires, l'obstruction de l'uretère, l'obstruction tumorale, ou des combinaisons de ceux-ci.

15. Le dispositif implantable tel que défini dans la revendication 7, qui comprend en outre une quelconque parmi une couche d'apprêt, une couche de contrôle de la libération, une couche supérieure, une couche de finition, ou une combinaison de celles-ci.

16. Le dispositif implantable de la revendication 7, dans lequel la couche de réservoir comprend un ou plusieurs agents bioactifs.

17. Le dispositif implantable de la revendication 16, dans lequel l'un ou les plusieurs agents bioactifs sont choisis du paclitaxel, du docétaxel, de l'estradiol, du 17-béta-estradiol, des donneurs d'oxyde nitrique, des superoxyde dismutases, des mimétiques de superoxyde dismutases, du 4-amino-2,2,6,6- tetraméthylpipéridine-1-oxyle (4-amino-TEMPO), du biolimus, du tacrolimus, de la déxaméthasone, de l'acétate de déxaméthasone, de la rapamycine, des dérivés de la rapamycine, de la 40-O-(2-hydroxy)éthyl-rapamycine (évérolimus), de la 40-O-(3-hydroxy)propyl-rapamycine, de la 40-O-[2-(2-hydroxy)éthoxy]éthyl-rapamycine, et de la 40-O-tétrazole-rapamycine, de la 40-epi-(N1-tétrazolyl)-rapamycine (ABT-578), du zotarolimus, de la 42-O-(2- éthoxyléthyl)rapamycine, du y-hiridun, du clobétasol, du pimécrolimus, du mésylate d'imatinib, de la midostaurine, du fenofibrate, et des combinaisons de ceux-ci.

18. Une couche comprenant un terpolymère statistique amorphe tel que défini dans la revendication 1, et un ou plusieurs agents bioactifs.
